(19) Europäisches Patentamt
European Patent Office
Office européen des brevets

(11) Veröffentlichungsnummer: **0 009 468**
**A1**

# (12) EUROPÄISCHE PATENTANMELDUNG

(21) Anmeldenummer: **79810101.0**

(22) Anmeldetag: **21.09.79**

(51) Int. Cl.³: **C 07 D 233/22,** A 01 N 43/50 // A61K31/415

(30) Priorität: **27.09.78 CH 10086/78**
**24.04.79 CH 3836/79**

(43) Veröffentlichungstag der Anmeldung: **02.04.80**
**Patentblatt 80/7**

(84) Benannte Vertragsstaaten: **BE CH DE FR GB IT NL**

(71) Anmelder: **CIBA-GEIGY AG, Patentabteilung Postfach, CH-4002 Basel (CH)**

(72) Erfinder: **Böger, Manfred, Lindenstrasse 33, D-7858 Weil am Rhein 5 (DE)**
Erfinder: **Drabek, Jozef, Dr., Benkenstrasse 12, CH-4104 Oberwil (CH)**
Erfinder: **Mattern, Günter, Dr., Sichternstrasse 35, CH-4410 Liestal (CH)**
Erfinder: **Traber, Walter, Dr., Neueneichweg 12, CH-4153 Reinach (CH)**

(54) **1-Alkylthio- und 1-Phenylthio-substituierte 2-(Phenoxyalkyl)-2-imidazoline, Verfahren zu ihrer Herstellung, Mittel welche diese Imidazoline enthalten und deren Verwendung zur Bekämpfung von Schädlingen der Ordnung Akarina.**

(57) Verbindungen der Formel I

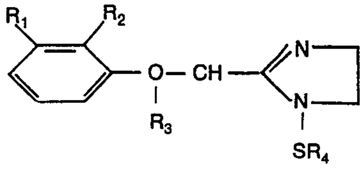

(I)

worin $R_1$ und $R_2$ je Chlor oder Methyl, $R_3$ Wasserstoff oder $C_1$–$C_4$-Alkyl und $R_4$ gegebenenfalls durch Cyan substituiertes $C_1$–$C_6$ -Alkyl oder gegebenenfalls durch Halogen und/oder Methyl substituiertes Phenyl bedeuten. Die Verbindungen der Formel I sind akarizid wirksam.

EP 0 009 468 A1

CIBA-GEIGY AG
Basel (Schweiz)

5-12050/1+2/+

1-Alkylthio- und 1-Phenylthio-substituierte 2-(Phenoxyalkyl)-2-imidazoline, Verfahren zu ihrer Herstellung, Mittel welche diese Imidazoline enthalten und deren Verwendung zur Bekämpfung von Schädlingen der Ordnung Akarina.

Die vorliegende Erfindung betrifft neue 1-Alkylthio- und 1-Phenylthio-substituierte 2-(Phenoxyalkyl)-2-imidazoline, welche eine Wirkung gegen Schädlinge besitzen, und Verfahren zu ihrer Herstellung sowie akarazide Mittel, welche die genannten Imidazoline als aktive Komponente enthalten, und Verfahren zur Bekämpfung von Schädlingen der Ordnung Akarina unter Verwendung der neuen Verbindungen. 1-Substituierte 2-(Phenoxyalkyl)-2-imidazoline mit pestizider, vor allem ektoparasitizider Wirkung sind bekannt (siehe z.B. japanische Patentveröffentlichung 76/106739 und DOS. 2,756,639). Nach vorliegender Erfindung werden neuartige Verbindungen dieses Typs bereitgestellt, die ebenfalls eine Wirkung gegen Schädlinge insbesondere gegen Vertreter der Ordnung Akarina aufweisen und welche aufgrund ihrer vorteilhaften biologischen Eigenschaften für die praktische Anwendung besonders geeignet sind.

Die erfindungsgemässen neuen 1-Alkylthio- und 1-Phenylthio-substituierte 2-(Phenoxyalkyl)-2-imidazoline entsprechen der Formel;

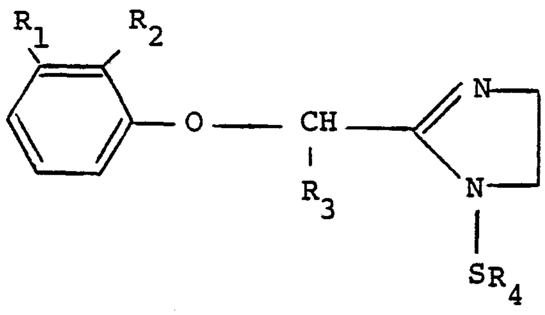

(I)

worin $R_1$ und $R_2$ je Chlor oder Methyl, $R_3$ Wasserstoff oder $C_1$-$C_4$-Alkyl und $R_4$ gegebenenfalls durch Cyan substituiertes $C_1$-$C_6$-Alkyl oder gegebenenfalls durch Halogen und/oder Methyl substituiertes Phenyl bedeuten.

Die für $R_3$ und $R_4$ in Frage kommenden Alkylgruppen sind die Methyl-, Aethyl-, n-Propyl-, i-Propyl-, n-Butyl, i-Butyl-, s-Butyl- und t-Butylgruppe sowie für $R_4$ die n-Pentyl- und n-Hexylgruppe und deren Isomere. Solche Alkylgruppen bilden ebenfalls das Alkylteil von substituierten Alkylgruppen.

In den Verbindungen der Formel I sind die folgenden Substituententypen sowie Kombinationen derselben untereinander bevorzugt:

1) Bei $R_3$: Wasserstoff und Aethyl insbesondere Aethyl; und

2) Bei $R_4$: 2-Cyan-prop-2-yl und gegebenenfalls ein- oder zweifach durch Halogen und/oder Methyl substituiertes Phenyl.

Erfindungsgemäss wurde nun überraschenderweise gefunden, dass die genannten Verbindungen der Formel I eine gegenüber ihrem aus der erwähnten DOS 2,756,639 bereits bekannten nächstliegenden Analogen, nämlich dem 1-Methylsulfonyl- und 1-Phenylsulfonyl-2-(2,3-dimethylphenoxymethyl)-2-imidazolin, überlegene Wirkung sowohl gegen pflanzenschädigende Akarinen (Milben z.B. der Familien Tetranychidae, Tarsonemidae, Eriophyidae, Tyroglyphidae und Glycyphagidae) als auch gegen ektoparasitäre Akariden (Milben und Zecken z.B. der Familien Ixodidae, Argasidae, Sarcoptidae und Dermanyssidae), welche an Nutztieren Schaden anrichten, aufweisen. Diesen Eigenschaften zufolge sind die Verbindungen der Formel I zur Bekämpfung von Schädlingen der Ordnung Akarina in Kulturen von Nutz- und Zierpflanzen sowie zur Bekämpfung von ektparasitären Zecken und Milben bei Nutztieren besonders geeignet.

Die Verbindungen der Formel I werden analog bekannten Verfahren hergestellt, in dem man z.B. eine Verbindung der Formel II

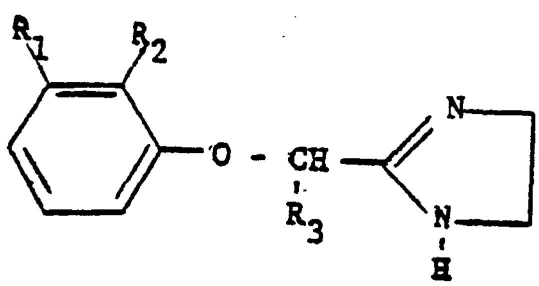

(II)

in Gegenwart einer Base mit einer Verbindung der Formel III

$$X - S - R_4 \qquad (III)$$

umsetzt, wobei in den Formeln II und III, $R_1$, $R_2$, $R_3$ und $R_4$ die unter Formel I bereits angegebenen Bedeutungen haben und X für ein Halogenatom insbesondere ein Chlor- oder Bromatom steht.

Das Verfahren wird zweckmässig bei einer Temperatur zwischen $-20^o$ und $30^oC$, bei normalem oder leicht erhöhtem Druck und vorzugsweise in Gegenwart eines gegenüber den Reaktionsteilnehmern inerten Lösungs- oder Verdünnungsmittels durchgeführt. Als Lösungs- oder Verdünnungsmittel eignen sich z.B. Aether und aetherartige Verbindungen wie Di-äthyläther, Di-isopropyläther, Dioxan und Tetrahydrofuran; aromatische Kohlenwasserstoffe wie Benzol, Toluol und Xylole; und Ketone wie Aceton, Methyläthylketon und Cyclohexanon.

Als für dieses Verfahren geeignete Basen kommen insbesondere tertiäre Amine, wie Trialkylamine, Pyridine und Dialkylaniline ferner Hydroxide, Oxide, Carbonate und Bicarbonate von Alkali- und Erdalkalimetallen sowie Alkalimetallalkoholate wie z.B. Kalium-tert. butylat und Natrium-methylat in Betracht.

Die Verbindungen der Formel I worin $R_3$ eine Alkylgruppe bedeutet liegen in Form von optisch aktiven Isomeren vor. Wenn deshalb bei der Herstellung keine optisch aktiven Ausgangsmaterialien verwendet werden, gelangt man zwangsläufig zu racemischen Gemischen. Solche Isomerengemische können z.B. mit Hilfe chromatographischen Trennmethoden

in die einzelnen isomeren Formen aufgetrennt werden. Unter dem Begriff der gegenwärtigen Erfindung versteht man sowohl die einzelnen optisch aktiven Isomere, als auch deren Gemische.

Die in dem vorstehend aufgeführten Verfahren verwendeten Ausgangsstoffe sind bekannt (vgl. D.O.S. 2,756,638) bzw. können analog den bekannten Methoden hergestellt werden.

Die Verbindungen der Formel I werden erfindungsgemäss als solche verwendet oder bilden einen Bestandteil von Mitteln, welche noch geeignete Trägerstoffe oder Zuschlagstoffe oder Gemische solcher Stoffe enthalten.

Geeignete Träger und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die akarizide Wirkung der erfindungsgemässen Mittel lässt sich durch Zusatz anderer Akarizide und/oder Insektizide wesentlich verbreitern.

Als Zusätze eignen sich z.B.: org. Phosphorverbindungen; Nitrophenole und deren Derivate; Formamidine; Harnstoffe; pyrethrinartige Verbindungen; Karbamate und chlorierte Kohlenwasserstoffe.

Die erfindungsgemässen Mittel können z.B. als Stäubemittel, Granulate, Dispersionen, Lösungen und Aufschlämmungen sowie als in Wasser dispergierbare Spritzpulver, Pasten, Emulsionen und Emulsionskonzentrate vorliegen und angewendet werden.

Der Gehalt an Wirkstoff (Verbindung der Formel I) in den oben beschriebenen Mitteln liegt zwischen 0,1 bis 95%, dabei ist zu erwähnen, dass bei der Applikation aus dem Flugzeug oder mittels anderer geeigneter Applikationsgeräte auch höhere Konzentrationen eingesetzt werden können.

Die Wirkstoffe der Formel I können beispielsweise wie folgt formuliert werden:

Emulsionskonzentrat I

| | |
|---|---|
| 20 Gew.-Teile | des obengenannten Wirkstoffes werden in |
| 70 Gew.-Teilen | Xylol gelöst und mit |
| 10 Gew.-Teilen | eines Emulgiermittels, bestehend aus einem Gemisch eines Arylphenylpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion. |

Emulsionskonzentrat II

| | |
|---|---|
| 5 bis max. 30 Gew.-Teile | Wirkstoff werden unter Rühren bei Zimmertemperatur in |
| 30 Gew.-Teilen | Dibutylphthalat |
| 10 Gew.-Teilen | Solvent 200 (niederviskoses hocharomat. Erdöldestillat) |
| 15 bis 35 Gew.-Teilen | Dutrex 238 FC (viskoses hocharomat. Erdöldestillat) gelöst und mit |

| | |
|---|---|
| 10 Gew.-Teilen | eines Emulgatorgemisches, bestehend aus Ricinusöl-polyglykoläther und dem Calciumsalz der Dodecylbenzolsulfonsäure, versetzt. Das so erhaltene Emulsionskonzentrat gibt in Wasser milchige Emulsionen. |

## Spritzpulver

| | |
|---|---|
| 5 bis 30 Gew.-Teile | des Wirkstoffes werden in einer Mischapparatur mit |
| 5 Gew.-Teilen | eines aufsaugenden Trägermaterials (Kieselsäure K 320 oder Wessalon S) und |
| 55 bis 80 Gew.-Teilen | eines Trägermaterials (Bolus alba oder Kaolin B 24) und einem Dispergiermittelgemisch, bestehend aus |
| 5 Gew.-Teilen | eines Na-lauryl-sulfonates und |
| 5 Gew.-Teilen | eines Alkyl-aryl-polyglykoläthers, intensiv vermischt. Diese Mischung wird auf einer Stifft- oder Luftstrahlmühle bis auf 5-15 µm gemahlen. Das so erhaltene Spritzpulver gibt in Wasser eine gute Suspension. |

## Pour-on-Lösung

| | |
|---|---|
| Wirksubstanz | 30,0 g |
| Natrium-dioctylsulfosuccinat | 3,0 g |
| Benzylalkohol | 48,0 g |
| Erdnussöl | 19,8 g |
| | 100,9 g = 100 ml |

Die Wirksubstanz wird in dem Benzylalkohol unter Rühren, eventuell auch unter leichtem Erwärmen, gelöst. Zu der Lösung wird das Natrium-dioctylsulfosuccinat und das Erdnussöl gegeben und unter Erwärmen und gründlichem Durchmischen gelöst.

Die nachfolgenden Beispiele dienen zur näheren Erläuterung der Erfindung:

Beispiel 1: Herstellung von 1-(2-Cyan-prop-2-ylthio)-2-[1-(2,3-dimethylphenoxy)-propyl]-2-imidazolin.

Zu einer Lösung von 6,97 g 2-[1-(2,3-Dimethylphenoxy)-propyl]-2-imidazolin der Formel

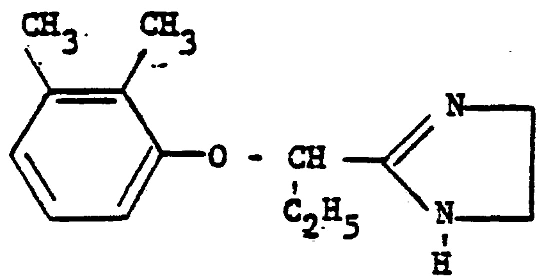

und 4 g Triäthylamin in 80 ml Toluol wurden 4,1 g α-(Chlorthio)-isobutyronitril unter ständigem Rühren und Kühlen bei 0° bis 10°C zugetropft und das erhaltene Reaktionsgemisch eine Stunde bei Raumtemperatur gerührt. Anschliessend wurde die Toluolphase mehrmals mit Wasser gewaschen. Nach dem Abdampfen des vorher getrockneten Toluols erhielt man das 1-(2-Cyan-prop-2-ylthio)-2-[1-(2,3-dimethylphenoxy)-propyl]-2-imidazolin der Formel

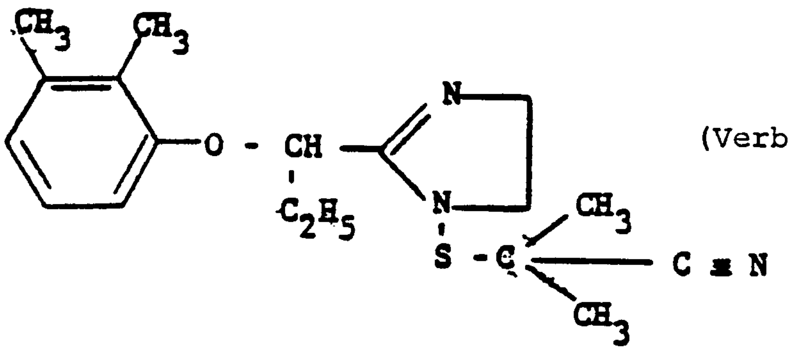

(Verbindung Nr. 1)

als ein gelbes viskoses Oel mit einem Smp. von 58-60°C.

Die folgenden Verbindungen der Formel I können analog zu den vorstehend beschriebenen Herstellungsverfahren hergestellt werden:

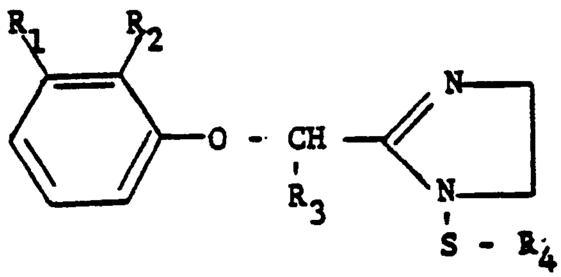

(I)

| Verb. Nr. | $R_1$ | $R_2$ | $R_3$ | $R_4$ | Physikalische Daten |
|---|---|---|---|---|---|
| 2 | $CH_3$ | $CH_3$ | $C_2H_5$ | $CH_3$ | |
| 3 | $CH_3$ | $CH_3$ | H | 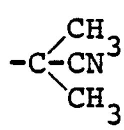 | Smp.: 77-79°C |
| 4 | Cl | $CH_3$ | $CH_3$ | 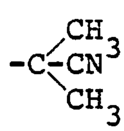 | $n_D^{20}$ 1,5554 |

| Verb. Nr. | $R_1$ | $R_2$ | $R_3$ | $R_4$ | Physikalische Daten |
|---|---|---|---|---|---|
| 5 | $CH_3$ | $CH_3$ | $n-C_4H_9$ | 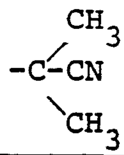 | |
| 6 | $CH_3$ | $CH_3$ | $C_2H_5$ | $n-C_6H_{13}$ | |
| 7 | $CH_3$ | $CH_3$ | H | 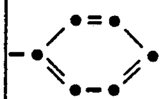 | Smp. 61-63°C |
| 8 | $CH_3$ | $CH_3$ | $n-C_3H_7$ | 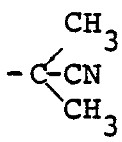 | $n_D^{20}$ 1,5403 |
| 9 | $CH_3$ | $CH_3$ | H | 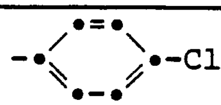 | Smp. 79-90°C |
| 10 | $CH_3$ | $CH_3$ | $C_2H_5$ | 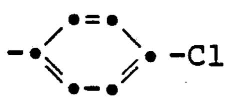 | $n_D^{20}$: 1,5939 |
| 11 | $CH_3$ | $CH_3$ | H | 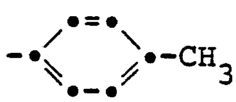 | Smp.: 79-81°C |
| 12 | $CH_3$ | $CH_3$ | $C_2H_5$ | 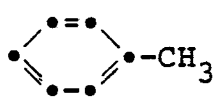 | $n_D^{20}$: 1,5838 |
| 13 | Cl | Cl | $C_2H_5$ | 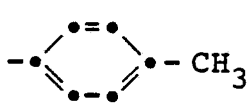 | $n_D^{20}$: 1,6035 |
| 14 | $CH_3$ | $CH_3$ | $C_2H_5$ | 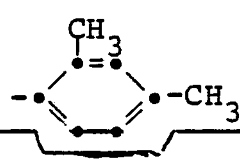 | Smp.: 67-69°C |
| 15 | $CH_3$ | $CH_3$ | H | 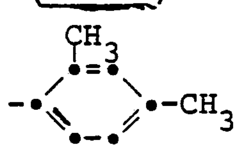 | $n_D^{20}$: 1,5818 |

**Beispiel 2: Wirkung gegen pflanzenschädigende Akariden (Milben): Tetranychus urticae (OP-sensible) und Tetranychus cinnabarinus (OP-tolerant).**

Die Primärblätter von Phaseolus vulgaris Pflanzen wurden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt. (Die Toleranz bezieht sich auf die Verträglichkeit von Diazinon.)

Die so behandelten infestierten Pflanzen wurden mit einer Versuchslösung enthaltend 400 oder 200 ppm der zu prüfenden Verbindung bis zur Tropfnässe besprüht.

Nach 24 Stunden und wiederum nach 7 Tagen wurden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25°C.

Verbindungen der Formel I zeigen in diesem Versuch eine positive Wirkung gegen Individuen der Spezies Tetranychus urticae und Tetranychus cinnabarinus.

Beispiel 3: Wirkung gegen ektoparasitäre Akariden (Zecken): Rhipicephalus bursa (Imagines und Larven), Amblyomma hebraeum (♀ Imagines, Nymphen und Larven) und Boophilus microplus (Larven-OP sensible und OP-tolerant).

Als Testobjekte wurden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die Testtiere wurden für kurze Zeit in eine wässrige Emulsion bzw. Lösung enthaltend 0,1; 1,0; 10; 50 oder 100 ppm. der zu prüfenden Verbindung getaucht.

Die in Teströhrchen befindlichen Emulsionen bzw. Lösungen wurden dann mit Watte aufgenommen und die benetzten Testtiere in den so kontaminierten Röhrchen belassen.

Eine Auswertung der erzielten Abtötungsrate bei jeder Konzentration erfolgte für Larven nach 3 Tagen und für Nymphen und Imagines nach 14 Tagen.

Verbindungen der Formel I zeigen in diesem Versuch eine gute Wirkung gegen Larven, Nymphen und Imagines der Spezies Rhipicephalus bursa und Amblyomma hebraeum sowie gegen Larven (OP-res. und OP-sens.) der Spezies Boophilus microplus.

Patentansprüche

1. Verbindungen der Formel I

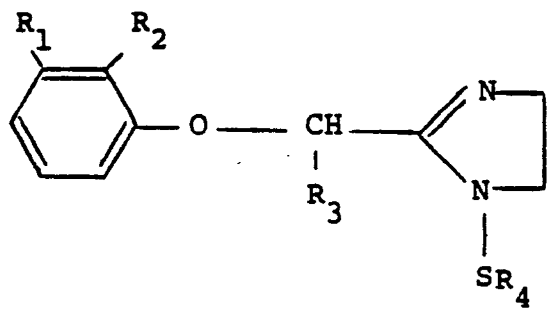

(I)

worin $R_1$ und $R_2$ je Chlor oder Methyl, $R_3$ Wasserstoff oder $C_1$-$C_4$-Alkyl und $R_4$ gegebenenfalls durch Cyan substituiertes $C_1$-$C_6$-Alkyl oder gegebenenfalls durch Halogen und/oder Methyl substituiertes Phenyl bedeuten.

2. Verbindungen nach Anspruch 1, worin $R_3$ Wasserstoff oder Aethyl bedeutet.

3. Verbindungen nach Anspruch 2, worin $R_3$ Aethyl bedeutet.

4. Verbindungen nach einem der Ansprüche 1 bis 3, worin $R_4$ 2-Cyano-prop-2-yl oder gegebenenfalls ein- oder zweifach durch Halogen und/oder Methyl substituiertes Phenyl bedeutet.

5. Verbindung nach Anspruch 4, der Formel

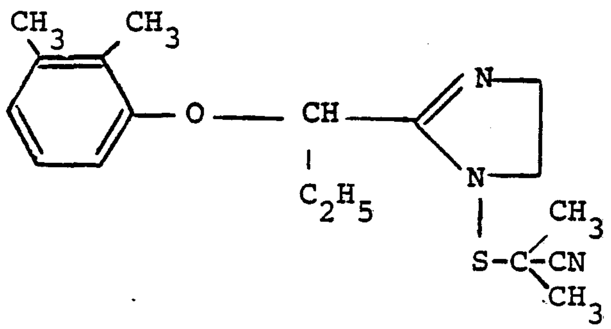

6. Verbindungen nach Anspruch 4, der Formel

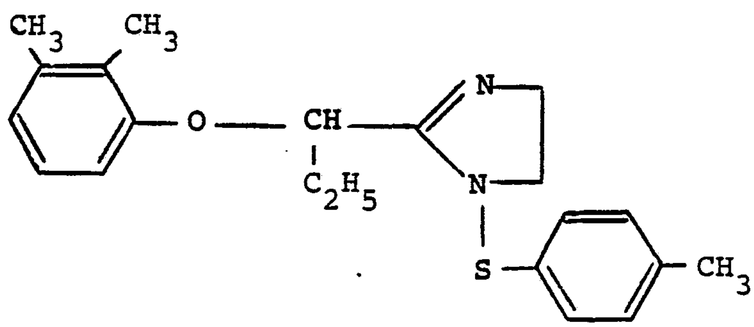

7. Verfahren zur Herstellung von in einem der Ansprüche 1 bis 6 definierten Verbindungen, dadurch gekennzeichnet, dass man eine Verbindung der Formel II

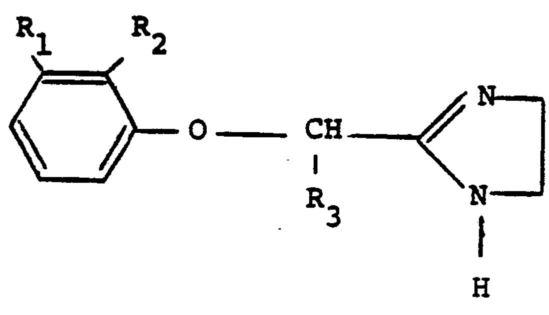

(II)

in Gegenwart einer Base mit einer Verbindung der Formel III

$$X - S - R_4 \qquad \text{(III)}$$

umsetzt, worin $R_1$, $R_2$, $R_3$ und $R_4$ die entsprechenden, in einem der Ansprüche 1 bis 6 angegebenen Bedeutungen haben

und Y ein Halogenatom bedeutet.

8. Akarizides Mittel enthaltend als aktive Komponente eine der in einem der Ansprüche 1 bis 6 definierten Verbindungen.

9. Verwendung von einer der in einem der Ansprüche 1 bis 6 definierten Verbindungen zur Bekämpfung von Schädlingen der Ordnung Akarina.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0009468

Nummer der Anmeldung
EP 79 81 0101

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 1 935 479 (NORDMARK-WERKE)<br>* Seiten 9 und 10 *<br>-- | 1,8 |
| P | DE - A - 2 818 367 (CIBA-GEIGY)<br>* Seiten 4-9 *<br>---- | 1,2 |

KLASSIFIKATION DER ANMELDUNG (Int. Cl.)

C 07 D 233/22
A 01 N 43/50//
A 61 K 31/415

RECHERCHIERTE SACHGEBIETE (Int. Cl.)

C 07 D 233/22
A 01 N 43/50
A 61 K 31/415

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8

Unvollständig recherchierte Patentansprüche:

Nicht recherchierte Patentansprüche: 9

Grund für die Beschränkung der Recherche: Verfahren zur chirurgischen oder therapeutischen Behandlung des menschlichen oder tierischen Körpers. (Siehe Art. 52(4) des Europäischen Patentübereinkommens)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 20-11-1979 | DE BUYSER |

EPA Form 1505.1 06.78